# EUROPEAN PATENT APPLICATION

(11) **EP 1 563 813 A2**
(43) Date of publication of application: **17.08.2005**
(21) Application number: 05000207.0
(22) Date of filing: 07.01.2005
(51) Int. Cl.: A61F 5/055

(54) **Cervical orthosis**

(30) Priority: 13.01.2004 US 756004
(71) Applicant: Spine Works, LLC, Traverse City, MI 49684 (US)
(72) Inventor: Farley, Daniel K., Traverse City, MI 49684 (US); Mulac, Anthony J., East Jordan, MI 49727 (US); Saunders, Richard L., Lebanon, NH 03766 (US)
(74) Representative: Oppermann, Ewald, Dipl.-Ing.

(57) **Abstract**

An external cervical fixation system includes a body configured to be secured to a patient, an attachment arm extending from the body, a bone screw, and a mounting fixture. The bone screw is mounted to the attachment arm, and has a threaded distal tip that is configured for threaded engagement with the patient's skull. The mounting fixture joins the attachment arm and the bone screw. The mounting fixture is configured to allow mounting and removal of the attachment arm and the bone screw when the bone screw is threadedly engaged with a patient's skull. The body can be adjusted independently of the bone screw.

## Description

### BACKGROUND OF THE INVENTION

The present invention relates to cervical fixation systems for use with patients with cervical or spinal injuries for immobilizing the neck of the patient to promote healing.

Cervical collars are orthopedic devices normally applied by medical practitioners, such as an orthopedic surgeon or neurosurgeon, for immobilizing a patient to promote healing which often takes many months. Collars immobilize the cervical spine by encasing the neck and chin in a rigid foam or foam lined plastic shell. These collars, however, may be overly confining and uncomfortable.

Conventional halo vests are also used to immobilize the patient. However, they are more rigid than the collar. The halos are typically comprised of a vest body having front and rear components for overlying the front and back of a patient's torso. The front and rear body components of the halo vest are normally secured to one another by flexible straps around the waist and over the shoulders. Halo support rods attach to the upper portions of the body components for supporting a halo that is secured to the patient's head. The halo is secured by a surgical procedure where four or more pins are driven into the patient's skull. The pins stay in place by using counter-pressure or opposing pin pressure. Around 8 pounds of pressure are applied to each pin thereby driving the pins into the outer surface of the skull and holding the device in place.

Conventional halos are too bulky and give patients claustrophobia. They are difficult to sleep in and difficult to maneuver (see, for example, U.S. Patent No. 5,261,873). Several problems exist with the use of conventional halo devices, including infection at the pin sites, loosening of the pins, and the resulting movement of the halo, penetration of the skull by the pins, unwanted loading of the halo caused by the shoulder straps which can be moved if the shoulders are elevated, and difficulty of adjustment.

A cervical fixation system that avoids the use of pins is disclosed in U.S. Patent No. 6,663,630. This design, while removing some of the problems of other systems, does not provide bone screws that can be located independently and placed before securing the fixation system to the body, thus limiting adjustability somewhat. Due to the wide variety of human skull and body shapes, adjustability is an important quality of cervical fixation systems.

It is therefore one object of the present invention to eliminate one or all of the problems associated with known halo and/or collar devices, including loosening problems associated with pins, movement of the device caused by shoulder movement, bulkiness, and adjustability limitations.

### BRIEF SUMMARY OF THE INVENTION

An external cervical fixation system is disclosed that includes a body configured to be secured to a patient, an attachment arm extending from the body, a bone screw, and a mounting fixture. The bone screw is mounted to the attachment arm, and has a threaded distal tip that is configured for threaded engagement with the patient's skull. The mounting fixture joins the attachment arm and the bone screw. The mounting fixture is configured to allow mounting and removal of the attachment arm and the bone screw when the bone screw is threadedly engaged with a patient's skull. The body can be adjusted independently of the bone screw.

The external cervical fixation system may comprise a plurality of attachment arms with each attachment arm being independently adjustable. Further, the threaded distal tip of the bone screw may include a tapered thread. Additionally, the bone screw may include a shoulder proximal to the threaded distal tip configured to prevent penetration through a skull. The mounting fixture may include a bone screw clamp that includes an opening accepting a mounting feature of the bone screw. The opening is adjustable to secure and release the bone screw.

An external cervical fixation system is disclosed that includes a body configured to be secured to a patient, a plurality of attachment arms, bone screws, and a plurality of mounting fixtures. The body includes a front assembly and a back assembly joined by sides. The independently adjustable attachment arms extend from the body. Bone screws are mounted to the attachment arms. Each of the bone screws has a threaded distal tip and a mounting feature. The threaded distal tip is configured for threaded engagement with a patient's skull. Each of the plurality of mounting fixtures joins one of the attachment arms to one of the bone screws. Each of the mounting fixtures includes a bone screw clamp that includes an opening that accepts a mounting feature of the bone screw. The opening is adjustable to secure and release the bone screw wherein the body can be adjusted independently of the bone screw.

The back assembly may include a back plate and a cross bar separately mounted to the sides. The back plate is configured to abut the back of a patient, and the attachment arms are adjustably mounted to the cross bar. Optionally, the sides may include a lateral plate made of a carbon fiber.

An external cervical fixation system is disclosed that includes a body configured to be secured to a patient, a plurality of independently adjustable attachment arms extending from the body, a halo type fixture adapted for mounting to a skull with skull pins, and a plurality of mounting fixtures. The halo type fixture is joined to the body by the adjustable attachment arms, and the attachment arms are joined to the halo by the mounting fixtures. The halo type fixture includes mounting features. The mounting fixtures include a clamp that has an opening that accepts a mounting feature of the halo. The opening is adjustable to secure and release the mounting feature.

### BRIEF DESCRIPTION OF THE SEVERAL VIEWS OF THE DRAWINGS

Figure 1 is a rear isometric view of a patient fitted with an external cervical fixation system formed in accordance with an embodiment of the present invention.

Figure 2 is a front isometric view of a patient fitted with an external cervical fixation system of Fig. 1.

Figure 3 is a rear view of the cervical fixation system of Fig. 1.

Figure 4 is a side view of the cervical fixation system of Fig. 1.

Figure 5 is a front view of the front assembly of the cervical fixation system of Fig. 1.

Figure 6 is a side view of a bone screw formed in accordance with an embodiment of the present invention.

Figure 7 is a rear view of the bone screw of Fig. 6.

Figure 8 is an exploded isometric view of the external cervical fixation system of Fig. 1.

Figure 9 is a top view of a mounting assembly for a cervical fixation system formed in accordance with another embodiment of the present invention.

Figure 10 is a rear view of the mounting assembly of Fig. 9.

Figure 11 is a side view of a bone screw for use with the mounting assembly of Fig. 9.

Figure 12 is a side view of a skull pin for use with the mounting assembly of Fig. 9.

Figure 13 is a side view of an external cervical fixation system formed in accordance with another embodiment of the present invention.

Figure 14 is a top view of a halo for use with the external cervical fixation system of Fig. 13.

### DETAILED DESCRIPTION

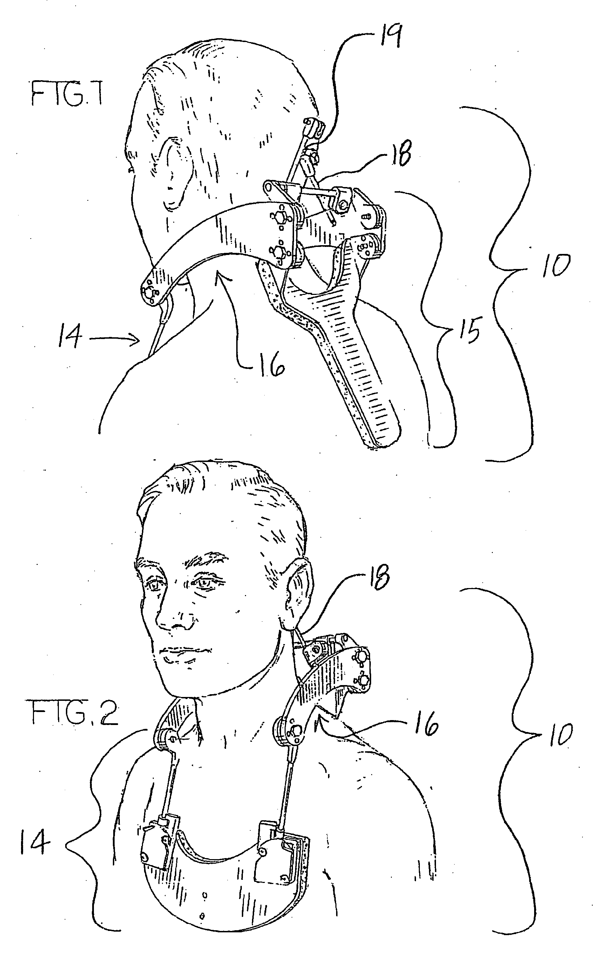

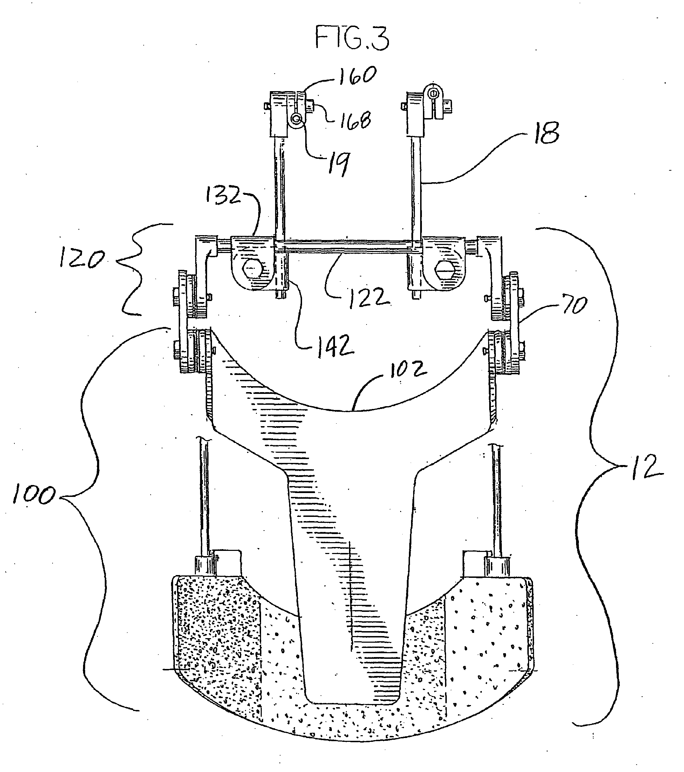

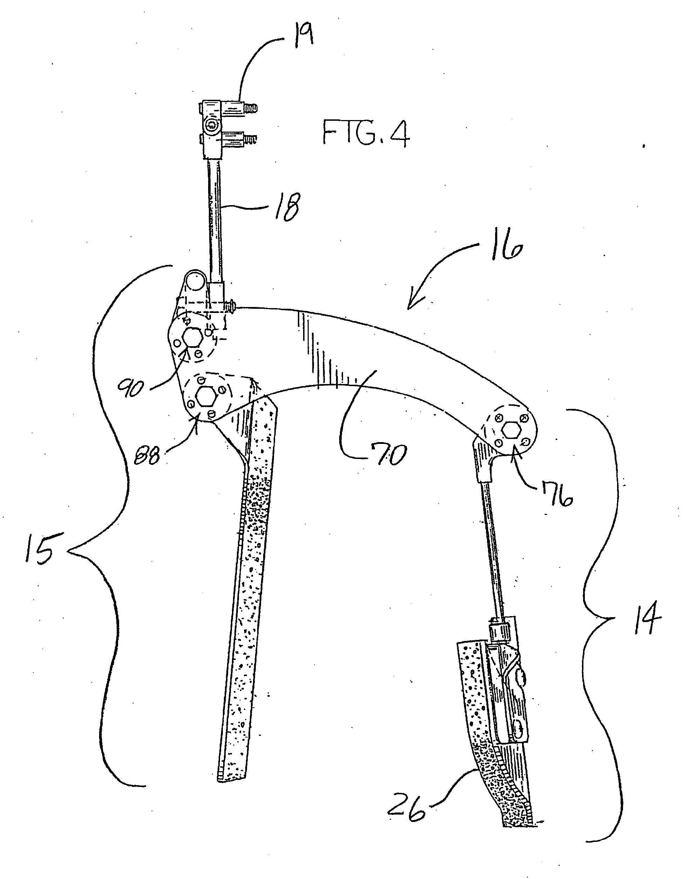

Figures 1 through 4 present different views of one embodiment of the present invention. Figures 1 and 2 provide rear and front isometric views, respectively, of a patient fitted with an external cervical fixation system 10. Figure 3 shows a rear view of the external cervical fixation system 10 and Figure 4 shows a side view of the external cervical fixation system 10. The cervical fixation system 10 holds the patient's skull in place relative to the upper body.

The cervical fixation system 10 includes a body 12 that includes a front assembly 14 and a back assembly 15 joined by sides 16, all rigidly interconnected. The front assembly 14, back assembly 15, and sides 16, along with associated hardware, form a rigid open frame that supports the patient's skull without encasing the patient's neck or requiring shoulder straps and/or a waist belt. Attachment arms 18 extend from the body 12. In an alternative embodiment, the attachment arms 18 extend from a vest similar to conventional designs. The attachment arms 18 are also joined to bone screws 19 that secure to the patient's skull.

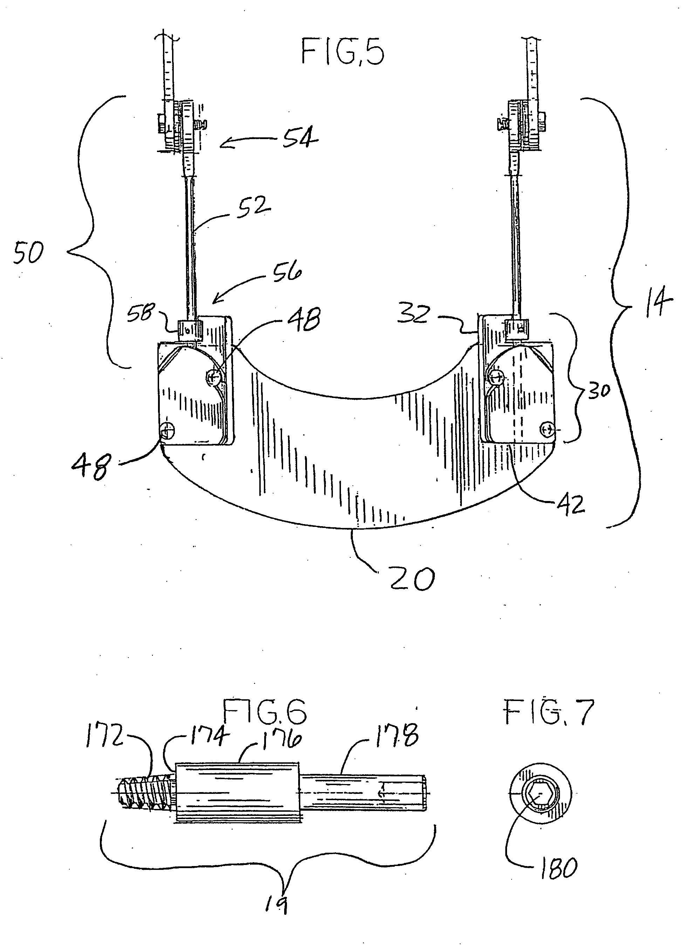

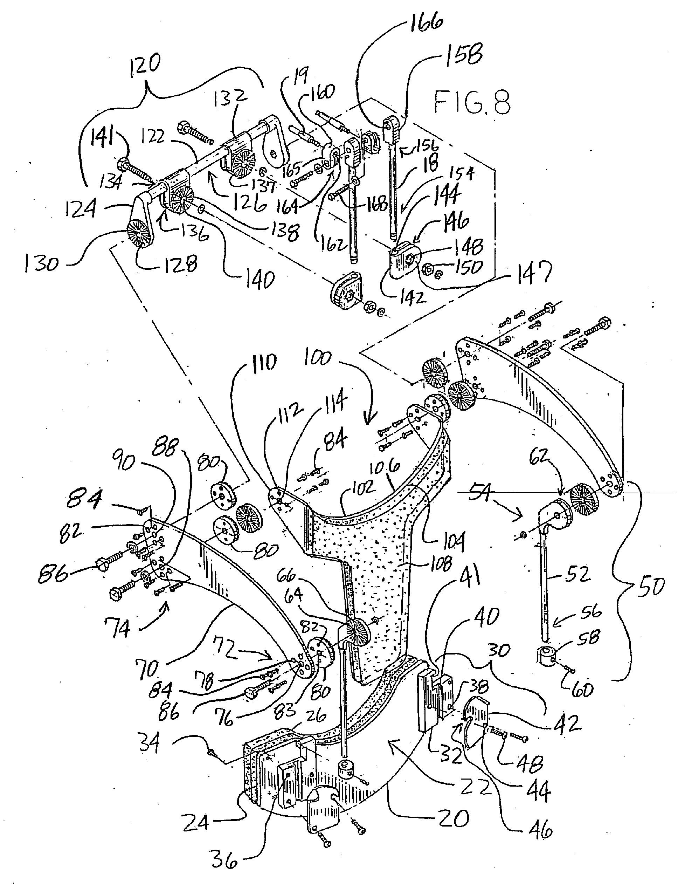

Figure 5 presents a front view of the front assembly 14 of the cervical fixation system 10, and Fig. 8 provides an exploded isometric view of the cervical fixation system 10. As best seen in Figs. 5 and 8, the front assembly 14 of the cervical fixation system 10 includes a front plate 20, a retention assembly 30, and a front arm assembly 50.

The front plate 20 includes a forward portion 22 and a rearward portion 24. The forward portion 22 faces away from the patient, while the rearward portion 24 faces toward the patient. Front padding 26 adheres to the rearward portion 24 to allow the front assembly 14 to be comfortably secured to the patient.

The retention assembly 30 is mounted to the front plate 20. The retention assembly 30 includes a retention base 32 and a retention clip 42. The retention base mounting screw 34 cooperates with the retention base mounting hole 36 of the retention base 32 to secure the retention base 32 to the front plate 20. The retention base 32 includes retention clip mounting holes 38. The retention base 32 also includes a channel 40 and a retention ledge 41. The channel 40 and retention ledge 41 allow the front arm assembly 50 to be mounted to the retention assembly 30 and held in place.

The retention clip 42 mounts to the retention base 32 to cover the channel 40. The retention clip 42 includes a retention clip through hole 44 and a retention clip slot 46. Retention clip mounting screws 48 mount through the retention clip through hole 44 and retention clip slot 46 into the retention clip mounting holes 38 of the retention base 32. To expose the channel 40 to either remove or insert a front arm assembly 50 into the retention assembly 30, the retention clip mounting screw 48 corresponding to the retention clip slot 46 is loosened, and the retention clip 42 may then be rotated to expose the channel 40.

The front arm assembly 50 includes a front arm 52, a retainer 58, and related hardware. The front arm 52 includes an upper portion 54 and a lower portion 56. The lower portion 56 is cylindrical and sized to be received by the channels 40 of the retention base 32. The retainer 58 receives the lower portion 56 of the front arm 52 and is held in place on the front arm 52 by a retainer set screw 60. The retainer 58 cooperates with the retention ledge 41 of the retention assembly 30 to hold the front arm assembly 50 in place. The front arm assembly 50 may be adjusted by loosening the retainer set screw 60 and repositioning the front arm assembly 50 by sliding the front arm 52 up or down in the channel 40. The retainer 58 acts to hold the front arm assembly 50 in place vertically, while the retention clip 42 and channel 40 cooperate to hold the front arm assembly 50 in place laterally. The upper portion 54 of the front arm 52 includes a mounting area 62. The mounting area 62 is a generally circular shaped flange extending from the front arm 52. The mounting area 62 includes a mounting hole 64 penetrating through it. The mounting hole 64 is a tapped, or threaded, hole. The mounting area 62 also includes a serrated surface 66 surrounding the mounting hole 64 and facing toward the sides 16.

The side 16, as best seen in Figs. 4 and 8, includes a lateral plate 70. The lateral plate 70 is preferably made of carbon fiber to allow for radio transparency during lateral X-rays. The carbon fiber is also light, thereby contributing to patient comfort. The lateral plate 70 includes a front portion 72 and a back portion 74. The front portion 72 is oriented toward the front of the patient, and the back portion 74 is oriented toward the back of a patient. The front portion 72 includes a front arm mounting through hole 76 and serrated washer mounting through holes 78. A serrated washer 80 is mounted to the front portion 72 of the lateral plate 70. The serrated washer 80 includes serrated washer mounting holes 82 located in a circle around a serrated washer through hole 83. The serrated washer mounting holes 82 receive serrated washer mounting screws 84 that also pass through the serrated washer mounting through holes 78 of the lateral plate 70. The serrated washer mounting screws 84 hold the serrated washer 80 secure to the lateral plate 70 and do not allow it to rotate relative to the lateral plate 70. The sides 16 also include a mounting screw 86 that passes through the front arm mounting through hole 76 as well as the serrated washer through hole 83 before engaging the threads of the mounting hole 64 of the front arm 52. The mounting screw 86 holds the lateral plate 70 secured to the front arm 52. The serrated face of the serrated washer 80 cooperates with the serrated surface 66 of the front arm 52 to prevent rotation between the sides 16 and the front assembly 14.

The back portion 74 of the lateral plate 70 includes a back plate mounting through hole 88 and a back cross bar mounting through hole 90. Serrated washer mounting holes 82, serrated washer mounting screws 84 and serrated washers 80 are also associated with each of the back plate mounting through hole 88 and the back cross bar mounting through hole 90.

As best seen in Figs. 1, 3, 4, and 8, the back assembly 15 includes a back plate assembly 100 and a back cross bar assembly 120. The back plate assembly 100 includes a back plate 102, padding 108, and related hardware. The back plate 102 includes a forward portion 104 that faces toward the patient and a rearward portion 106 that faces away from the patient. Padding 108 is mounted to the forward portion 104 to help hold the back plate 102 comfortably and securely to the back of a patient.

Extending away from the patient from the rearward portion 106 of the back plate 102 are the mounting portions 110. The mounting portions 110 include serrated washer mounting through holes 112 that accept serrated washer mounting screws 84 to hold a serrated washer 80 secure to the mounting portions 110 of the back plate 102. The mounting portions 110 also include a mounting hole 114. The mounting hole 114 is tapped, or threaded, and accepts a mounting screw 86. The mounting screw 86 is accepted by the back plate mounting through hole 88 of the lateral plate 70, the serrated washer through hole 83 of the serrated washers 80 that are associated with the lateral plate 70 and the back plate assembly 100, and the mounting hole 114 of the back plate assembly 100. The mounting screw 86 holds the lateral plate 70 securely to the mounting portion 110 of the back plate 102. The serrated faces of the serrated washers 80 are oriented toward each other and cooperate to prevent the lateral plate 70 from rotating relative to the back plate 102.

The back cross bar assembly 120 includes a back cross bar 122 and cross bar clamps 132. The back cross bar 122 includes an end portion 124 and a middle portion 126. The end portion 124 of the back cross bar assembly facilitates mounting to the sides 16. The end portion 124 may be removably mounted to the back cross bar 122 with a set screw to allow for mounting and removal of cross bar clamps 132 to the back cross bar 122. The end portion 124 includes a mounting hole 130. The mounting hole 130 is a threaded hole that accepts a mounting screw 86. The mounting hole 130 is surrounded by a serrated surface 128 that faces toward the sides 16. A mounting screw 86 passes through the back cross bar mounting through hole 90 of the lateral plate 70 (as well as a serrated washer through hole 83 of a serrated washer 80) and is accepted by the mounting hole 130 of the back cross bar 122 to secure the back cross bar assembly 120 to the sides 16. The serrated surface 128 of the back cross bar 122 cooperates with the serrated surface of the serrated washer 80 to prevent the back cross bar assembly 120 from rotating relative to the lateral plate 70.

The middle portion 126 is a generally cylindrical section extending between the two end portions 124. The cross bar clamps 132 are mounted to the back cross bar 122 at the middle portion 126. The cross bar clamps 132 include an opening 134 that accepts the middle portion 126 of the back cross bar 122. Connected to and extending from the opening 134 is a gap 136 separating wings 137. Bringing the wings 137 together reduces the gap 136 and the opening 134, thereby securing the cross bar clamp 132 rigidly to the middle portion 126 of the back cross bar 122. The cross bar clamps 132 also include a serrated surface 138, surrounding a mounting hole 140. The mounting hole 140 may have a hexagonal countersink (not shown) to accept the head of an upright bar mounting screw 141. The mounting hole 140 accepts the upright bar mounting screw 141.

The attachment arms 18 are secured to the cross bar clamps 132 of the back cross bar assembly 120 by upright bar clamps 142. Each upright bar clamp 142 has an opening 144 that accepts an adjustable attachment arm 18. Extending from the opening 144 is a gap 146 separating wings 147. The wings 147 may be brought together to reduce the gap 146 and the opening 144 to hold the adjustable attachment arm 18 securely within the upright bar clamp 142. The upright bar clamp 142 also includes a mounting hole 148 and a hex nut 150, as well as a serrated surface (not shown) that cooperates with the serrated surface 138 of the cross bar clamp 132. The mounting hole 148 and hex nut 150 accept the upright bar mounting screw 141. Tightening the hex nut 150 on the upright bar mounting screw 141 reduces both the gap 146 of the upright bar clamp 142 and the gap 136 of the cross bar clamp 132, thereby securing both clamps in place and securing the attachment arm 18 to the back cross bar assembly 120.

The adjustable attachment arm 18 includes a lower portion 154 and an upper portion 156. The lower portion 154 is generally cylindrical and sized to be loosely accepted by the opening 144 of the upright bar clamp 142 when the wings of the 147 of the upright bar clamp 142 have not been brought together by the upright bar mounting screw 141.

The upper portion 156 includes a screw clamp mounting portion 158 including a mounting hole 166. A screw clamp 160 corresponds to the screw clamp mounting portion 158 and includes an opening 162, a gap 164, and wings 165. The structure of the screw clamp 160 is similar to the upright bar clamp 142 and the cross bar clamps 132. The screw clamp 160 helps secure the bone screw 19 to attachment arm 18.

As can be seen in Figs. 6 and 7, the bone screw 19 includes a threaded distal portion 172, an intermediate portion 176, and a mounting portion 178. The threaded distal portion 172 includes a tapered thread that is configured to engage a patient's skull. The tapered thread may be self-tapping.

Pins used with conventional halos only restrict movement along their axes in one direction. Consequently, a number of pins are required to hold a conventional halo in place using counter pressure or opposing pin pressure. This results in the bulkiness of halos and invites problems with loosening pins. Use of the bone screw 19 with a threaded distal portion 172 that engages the human skull avoids those problems by providing a rigid attachment to the skull, thereby eliminating the need for the use of counter pressure, and allowing for attachment at a lower number of required locations along the skull.

The threaded distal portion 172 of the bone screw 19 abuts the intermediate portion 176. The threaded distal portion 172 joins the intermediate portion 176 at a shoulder 174. The shoulder 174 helps to prevent penetration of the skull by the threaded distal portion 172. The mounting portion 178 abuts the intermediate portion 176 opposite the threaded distal portion 172. The mounting portion 178 is a generally cylindrical portion sized to be accepted by the opening 162 of the screw clamp 160. The mounting portion 178 includes a hexagonal countersink 180 that facilitates driving the bone screw 19 with an allen wrench.

To position the cervical fixation system 10 on a patient, first the bone screws 19 may be placed. Screw cites are selected and marked on a patient's skull. Once the screw cites have been marked, test holes should be drilled to determine the thickness of the skull. Once the thickness of the skull has been determined, a bone screw 19 with an appropriately sized distal tip 172 may be secured to the patient's skull using a 25 pound torque wrench. Next the body 12 of the cervical fixation system 10 may be secured to the patient's upper body. The front assembly 14, the back assembly 15, and sides 16 should be loosely connected to each other. Once the final position of the body is determined, the mounting screws 86 that hold the various components of the body 12 together may be tightened, providing a secure rigid fit to the patient. With the adjustable attachment arms 18 loosely attached to the back cross bar 122, the screw clamp 160 may be positioned and secured to the bone screw 19 and the attachment arm 18. With the patient's skull positioned in the desired location relative to the body 12, the upright bar mounting screw 141 may be tightened in the hex nut 150 thereby tightening the cross bar clamp 132 and the upright bar clamp 142 to securely hold the adjustable attachment arm 18 in place.

The use of adjustable attachment arms 18 allows the bone screws 19 to be positioned before the body 12 is positioned. This allows for precise location of the bone screws 19 in a particular patient's skull. The adjustable attachment arms 18 and screw clamps 160 allow the body 12 to be positioned independently of the bone screws 19. This provides a high degree of adjustability maximizing patient convenience and comfort. The cervical fixation system 10 also provides a low profile so that the patient can lie down on the external cervical fixation system 10. The front assembly 14, the back assembly 15, and the sides 16 cooperate to form a body 12 that is an open frame that avoids the uncomfortableness and claustrophobia caused by halos and collars that encapsulate a patient's skull, neck, and chin.

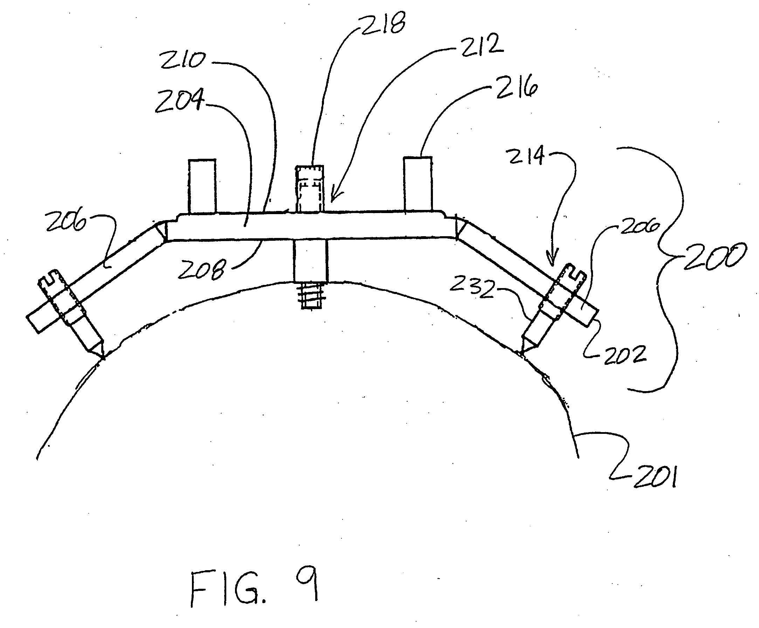

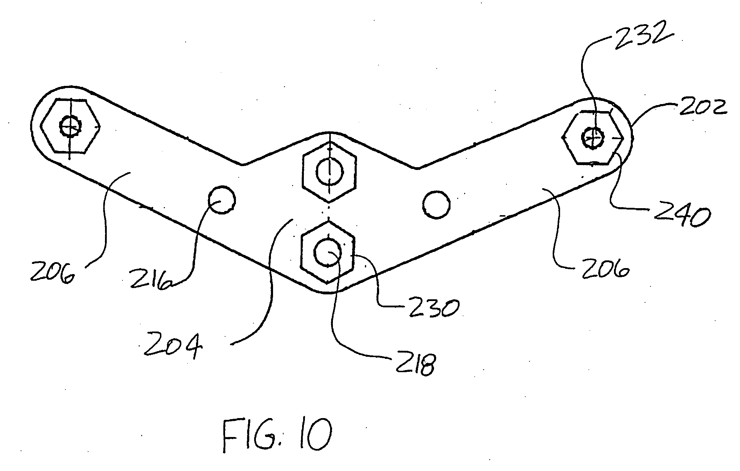

Figures 9-12 illustrate portions of another embodiment of the present invention that allows the body of a cervical fixation system to be positioned independently of bone screws. Figures 9 and 10 provide top and rear views, respectively, of a mounting assembly 200 that provides alternate mounting to the skull from the previously described embodiment. The mounting assembly 200 includes a mounting plate 202, bone screws 218, bone screw nuts 230, skull pins 232, and skull pin nuts 240.

As shown in Figs. 9-10, the mounting plate 202 includes a front 208 and a back 210. The front 208 is oriented toward the skull 201 to which the mounting plate 202 is adapted to be mounted, and the back 210 is oriented away from the skull 201. The mounting plate 202 also includes wings 206 extending from a middle portion 204. The wings 206 extend laterally outward from the middle portion 204 and bend toward the front of the patient.

The middle portion 204 includes screw holes 212 and mounting posts 216. The screw holes 212 are sized to accept the bone screws 218. The screw holes 212 have a counter bore (not shown) to provide proper seating of the bone screws 218 in the screw holes 212, and bone screw nuts 230 secure the bone screws 218 to the mounting plate 202 (as shown in Fig. 10). The mounting posts 216 extend from the back 210 of the mounting plate 202. Each mounting post 216 is generally cylindrical and sized to be accepted by an opening of a clamp, such as the screw clamps 160 (see Figs. 3 and 8), which attaches the mounting plate 202 to an attachment arm 18 (see Figs. 3 and 8).

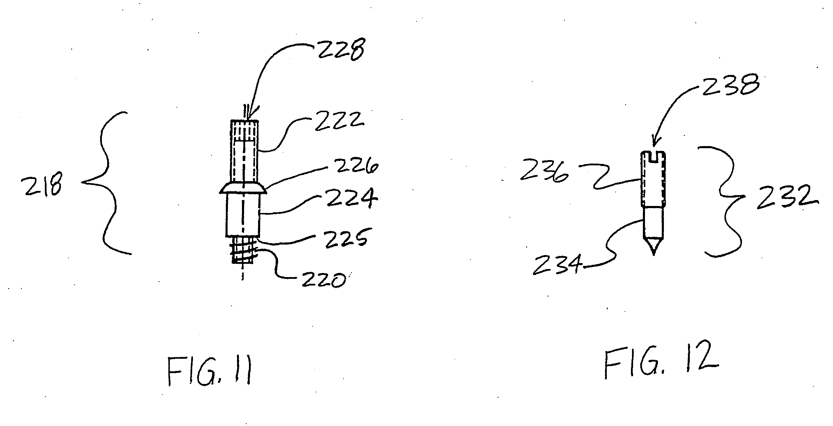

Fig. 11 shows a side view of the bone screw 218 that is accepted by the screw hole 212 and the bone screw nut 230 to secure the mounting plate 202 to the skull 201. The bone screw 218 includes a first threaded portion 220 and a second threaded portion 222 joined by an intermediate portion 224. The first threaded portion 220 is a tapered thread that threadingly engages the skull 201. The first threaded portion 220 joins the intermediate portion 224 at a shoulder 225. The shoulder 225 is sized and located to abut the outer surface of a patient's skull 201 (Fig. 9) to prevent the first threaded portion 224 from penetrating too deeply through the skull 201. The bone screw 218 also includes a flange 226 extending from the intermediate portion 224. The flange 226 is accepted by a counterbore of the screw hole 212 of the mounting plate 202 and acts to position the mounting plate 202 properly relative to the bone screw 218 and the skull 201. The second threaded portion 222 is a straight thread that is accepted by the bone screw nut 230 (Fig. 10). A hex counterbore 228 extends into the second threaded portion 222 to allow a surgeon to turn the bone screw 218.

Returning to Figs. 9-10, the mounting plate 202 also includes pin holes 214 extending through the wings 206. The pin holes 214 are threaded and accept the skull pins 232. The skull pins 232 act to stabilize the mounting plate 202 and prevent rocking of the mounting plate 202. Once a skull pin 232 has been adjusted to its desired position, a skull pin nut 240 is attached to secure the skull pin 232 in place.

Figure 12 illustrates a side view of the skull pin 232. The skull pin 232 includes a pin head 234, a threaded body 236, and a slot 238. The pin head 234 is oriented toward the skull 201. The threaded body 236 is sized to be threadedly engaged by both the pin hole 214 of the mounting plate 202 and the skull pin nut 240. The slot 238 extends into the threaded body 236 and allows the skull pin 232 to be turned with a screwdriver.

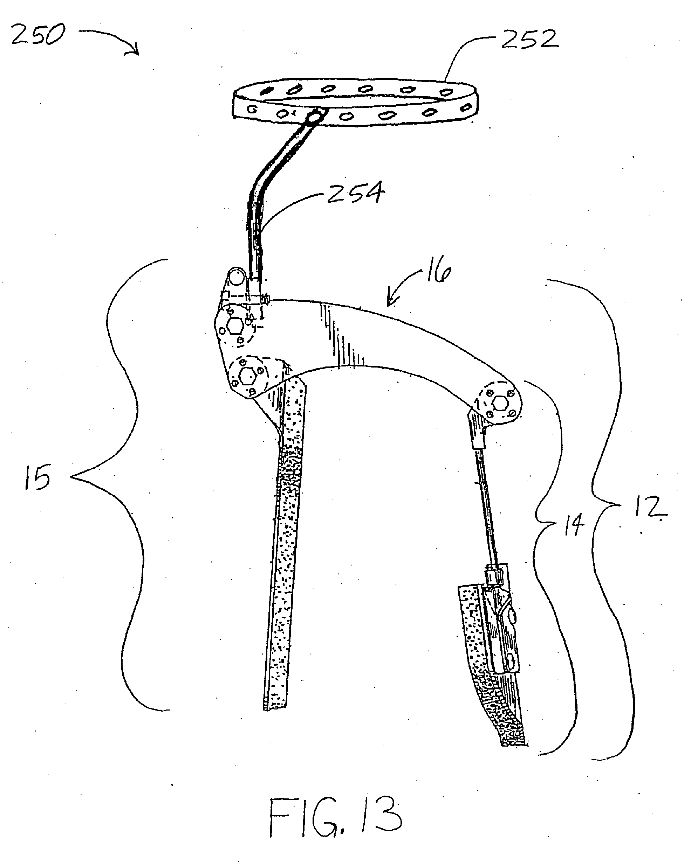

Figures 13-14 illustrate portions of another embodiment of the present invention. Figure 13 provides a side view of a cervical fixation system 150 that uses pins to mount to a patient's skull yet provides greater adjustability than conventional halo systems. The cervical fixation system 250 includes a halo 252 joined by attachment arms 254 to a body 12. Similar to an earlier described embodiment, the body 12 of the cervical fixation system 50 includes a front assembly 14 and a back assembly 15 joined by sides 16, all rigidly interconnected (see Figs. 1-4).

Returning to Fig. 13, the attachment arms 254 extend from the body 12, to which they are mounted. The attachment arms 254 are mounted to the back cross bar assembly 120 of the back assembly 15 of the body 12 by cross bar clamps 132 and upright bar clamps 142 (see Figs. 3 and 8). The attachment arms 254 are mounted to the halo 252 by screw clamps 160 (see Figs. 3 and 8).

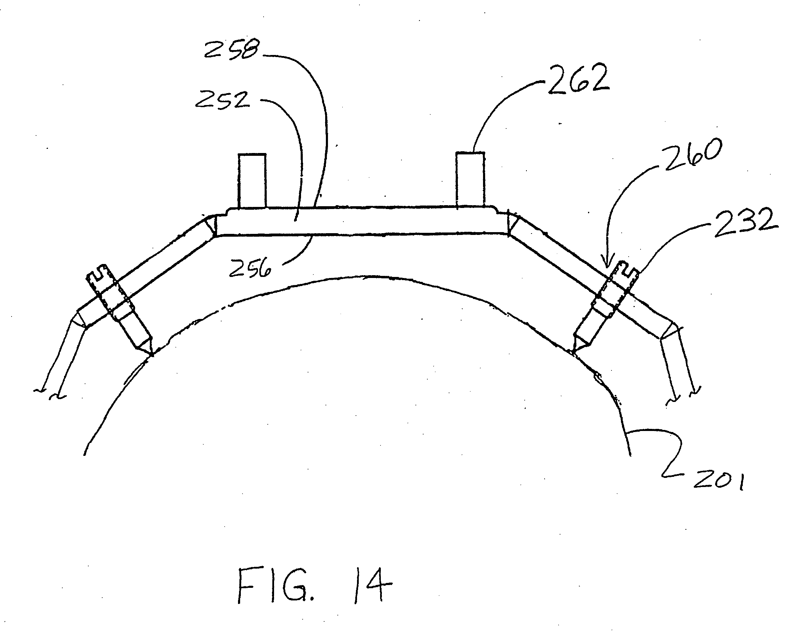

Figure 14 illustrates a top view of the halo 252 attached to a skull 201. The halo 252 is generally circular shaped and surrounds the skull 201. In alternative embodiments, the halo may be "U"-shaped or have open portions instead of completely encircling the skull. The halo 252 includes an inner portion 256 and an outer portion 258. The inner portion 256 faces toward the skull 201 when the halo 252 is mounted, while the outer portion 258 faces away. The halo 252 includes pin holes 260 spaced around the perimeter of the halo 252 and extending through the halo 252. The pin holes 260 are threaded and accept skull pins 232 that are placed around the periphery of the skull 201 to hold the halo 252 secure to the skull.

The halo 252 also includes mounting posts 262. The mounting posts 262 extend from the outer portion 258 of the halo 252. Each mounting post 262 is generally cylindrical and sized to be accepted by an opening of a clamp, such as a screw clamp 160 (see Figs. 3 and 8), which attaches the halo 252 to an attachment arm 254.

While particular embodiments of the invention have been shown, it will be understood that the invention is not limited thereto since modifications may be made by those skilled in the art, particularly in light of the foregoing teaching. It is therefore, the appended claims that define the true spirit and scope of the invention.

## Claims

1. An external cervical fixation system comprising
a body configured to be secured to a patient;
an attachment arm extending from said body;
a bone screw mounted to said attachment arm, said bone screw having a threaded distal tip configured for threaded engagement with a patient's skull; and
a mounting fixture joining said attachment arm and said bone screw, said mounting fixture configured to allow mounting and removal of said attachment arm and said bone screw when said bone screw is threadedly engaged, wherein said body can be adjusted independently of said bone screw.

2. The external cervical fixation system of claim 1 further comprising a plurality of said attachment arms, wherein said attachment arms are independently adjustable.

3. The external cervical fixation system of claim 1 wherein said threaded distal tip of said bone screw includes a tapered thread.

4. The external cervical fixation system of claim 1 wherein said bone screw includes a shoulder proximal to said threaded distal tip configured to prevent penetration of said threaded distal tip through a skull.

5. The external cervical fixation system of claim 1 wherein said bone screw includes a mounting feature and said mounting fixture includes a bone screw clamp, said bone screw clamp including an opening that accepts said mounting feature, said opening being adjustable to secure and release said bone screw.

6. The external cervical fixation system of claim 1 further comprising a mounting plate, said mounting plate including a mounting post and a screw hole, said screw hole accepting said bone screw, said mounting fixture including a bone screw clamp, said bone screw clamp including an opening that accepts said mounting post, said opening being adjustable to secure and release said mounting post.

7. The external cervical fixation system of claim 1 further comprising a mounting plate and a plurality of skull pins, said bone screw and said skull pins mounted to said mounting plate.

8. An external cervical fixation system comprising
a body configured to be secured to a patient;
a plurality of independently adjustable attachment arms extending from said body;
bone screws mounted to said attachment arms, each of said bone screws having a threaded distal tip and a mounting feature, said threaded distal tip being configured for threaded engagement with a patient's skull; and
a plurality of mounting fixtures, each of said mounting fixtures joining one of said attachment arms to one of said bone screws, each of said mounting fixtures comprising a bone screw clamp, said bone screw clamp comprising an opening that accepts said mounting feature of said bone screw, said opening being adjustable to secure and release said bone screw, wherein said body can be adjusted independently of said bone screw.

9. The external cervical fixation system of claim 8 wherein said threaded distal tip of said bone screw includes a tapered thread.

10. The external cervical fixation system of claim 8 wherein said bone screw includes a shoulder proximal to said threaded distal tip configured to prevent penetration of said threaded distal tip through a skull.

11. The external cervical fixation system of claim 8 wherein said bone screw includes an intermediate portion interposed between said threaded distal tip and said mounting feature, said intermediate portion including a shoulder proximal to said threaded distal tip configured to prevent penetration of said threaded distal tip through a skull.

12. An external cervical fixation system comprising
a body configured to be secured to a patient, said body including a front assembly and a back assembly rigidly joined by sides;
a plurality of independently adjustable attachment arms extending from said body;
bone screws mounted to said attachment arms, each of said bone screws having a threaded distal tip and a mounting feature, said threaded distal tip being configured for threaded engagement with a patient's skull; and
a plurality of mounting fixtures, each of said mounting fixtures joining one of said attachment arms to one of said bone screws, each of said mounting fixtures including a bone screw clamp, said bone screw clamp including an opening that accepts said mounting feature of one of said bone screws, said opening being adjustable to secure and release said bone screw, wherein said body can be adjusted independently of said bone screws.

13. The external cervical fixation system of claim 12 wherein said attachment arms extend from said back assembly of said body.

14. The external cervical fixation system of claim 12 wherein said back assembly includes a back plate and a cross bar separately mounted to said sides, said back plate configured to abut the back of a patient, said attachment arms adjustably mounted to said cross bar.

15. The external cervical fixation system of claim 12 wherein said threaded distal tip of said bone screw includes a tapered thread.

16. The external cervical fixation system of claim 12 wherein said sides include a lateral plate made of a carbon fiber.

17. The external cervical fixation system of claim 12 wherein said bone screw includes a shoulder proximal to said threaded distal tip configured to prevent penetration of said threaded distal tip through a skull.

18. The external cervical fixation system of claim 12 wherein said bone screw comprises an intermediate portion interposed between said threaded distal tip and said mounting feature, said intermediate portion including a shoulder proximal to said threaded distal tip configured to prevent penetration through a skull.

19. An external cervical fixation system comprising
a body configured to be secured to a patient, said body including a front assembly and a back assembly rigidly joined by sides;
a plurality of independently adjustable attachment arms extending from said body;
a halo type fixture adapted for mounting to a human skull with skull pins, said halo joined to said body by said adjustable attachment arms, said halo including a mounting feature; and
a plurality of mounting fixtures, each of said mounting fixtures joining one of said attachment arms to said halo, each of said mounting fixtures including a clamp, said clamp including an opening that accepts said mounting feature of said halo, said opening being adjustable to secure and release said mounting feature.

20. The external cervical fixation system of claim 19 wherein said back assembly includes a back plate and a cross bar separately mounted to said sides, said back plate configured to abut the back of a patient, said attachment arms adjustably mounted to said cross bar.
